# EUROPEAN PATENT APPLICATION

(11) **EP 4 623 934 A1**
(43) Date of publication of application: **01.10.2025**
(21) Application number: 23893623.1
(22) Date of filing: 07.11.2023
(51) Int. Cl.: A61K 47/64, A61K 38/40, A61K 38/28, A61K 38/29, A61K 38/27, A61K 31/7048, A61P 3/10

(54) **RECOMBINANT HUMAN SERUM ALBUMIN-DRUG CONJUGATE**

(30) Priority: 24.11.2022 CN 202211485727
(71) Applicant: Wuhan Healthgen Biotechnology Corp, Wuhan, Hubei 430020 (CN)
(72) Inventor: DONG, Liangliang, Wuhan, Hubei 430020 (CN); YANG, Daichang, Wuhan, Hubei 430020 (CN); XIA, Jun, Wuhan, Hubei 430020 (CN); CHEN, Rong, Wuhan, Hubei 430020 (CN); SHI, Chuan, Wuhan, Hubei 430020 (CN)
(74) Representative: Plougmann Vingtoft a/s
(86) International application number: PCT/CN2023/130068
(87) International publication number: WO 2024/109532

(57) **Abstract**

The present invention relates to a drug conjugate of recombinant human serum albumin and a therapeutic drug. The drug conjugate is formed by covalent coupling a therapeutic drug to a free sulfhydryl group at position 34 of recombinant human serum albumin via a heterobifunctional linker. The drug conjugate of the present invention not only enhances the pharmacological action and improves the pharmacokinetics of the therapeutic drug, but also significantly increases the coupling efficiency of the therapeutic drug to human serum albumin, thereby reducing the production cost of the drug conjugate.

## Description

### RELATED APPLICATIONS

This PCT Patent Application claims the benefit of priority under Patent Cooperation Treaty of Chinese Patent Application No. 202211485727.4, filed on November 24, 2022. The aforementioned application is expressly incorporated herein by reference in its entirety and for all purposes.

### TECHNICAL FIELD

The present invention generally related to biopharmaceuticals, and particularly provides to a drug conjugate based on recombinant human serum albumin and a therapeutic drug.

### BACKGROUND

The therapeutic efficacy of a drug depends on its availability at the target site with a specific concentration and dosing frequency, thus it is very important to maximize the therapeutic effects and minimize side effects for the patients in new drug development. Drugs such as proteins and polypeptides are basically completely inactivated after oral administration because they cannot tolerate the destruction of the digestive system, thus requiring larger doses. Meanwhile, drugs with small molecular weights are easily cleared by the kidneys or hydrolyzed by proteases even when injected, resulting in a short half-life in the body and requiring frequent administration. Conventional small molecule drugs are usually distributed non-specifically in the body, which results in low bio-availability at the target site in that to require high dose; meanwhile, due to their small molecular weights to rapid clearance by the kidneys, these need to be administered frequently. The conventional administration method and frequency cause more inconvenience and painful to patients.

Human Serum Albumin (HSA) is a most abundant protein in human plasma, accounting for more than 60% of total serum protein. HSA has a half-life of approximately 19 days. It consists of 585 amino acids, including 17 disulfide bonds and a free cysteine at position 34 (Cys34) with a molecular weight of approximately 66.5 kDa. HSA not only has the physiological function of maintaining plasma osmolarity, but also is an important non-specific transport protein in the body, which can reversibly bind to many insoluble organic small molecules or inorganic ions in the body to form the soluble complexes, which become the transport form of these substances in the blood circulation.

In the physiological environment, HSA exists as a negative charge and each HSA molecule is capable of carrying more than 200 negative charges, avoiding to be phagocytosed by the cells of the endothelial system. Meanwhile, it is very stable in the blood since it can disperse inside and outside the blood vessels. The characters of long half-life of HSA is attribute to a recycling mechanism of FcRn receptor-mediated (pH-dependent, preventing degradation by the lysosomal pathway) and to its ability to avoid renal clearance due to HSA has a large molecular weight than the renal threshold and can be recycled by the receptor-mediated endocytosis in the renal proximal tubule, thus avoiding renal clearance.

HSA also is tumor- and inflammation-targeted. The increased vascular permeability and poor lymphatic drainage at the tumor or inflammatory site makes drug molecules easier to permeate out of the tumor or inflammation vessels, and difficult to reenter the body circulation through the lymphatic route leading accumulation in the tumor or inflammation tissue. This phenomenon is known as enhanced permeability and retention effect (EPR). The concentration of HSA in the blood is about 45-55 mg/ml, while is about 14 mg/ml in the tumor stroma. At this concentration difference, HSA shows good passive tumor targeting through the EPR effect. In addition, HSA is dependent on the lymphatic system to circulation from the extracellular space, making it easily to accumulate in tumors with poor lymphatic drainage. Furthermore, the rapid metabolism and growth of cells at the tumor or inflammatory site requires actively uptake large amounts of extracellular proteins as a source of amino acids including HSA, which makes HSA more likely to be distributed in tumor or inflammatory tissues. MTX-HSA is the first chemical conjugate of HSA to be applied in clinical practice and is derived from the covalent coupling of methotrexate (MTX) to the lysine residue of HSA. Compared to MTX, data show that MTX-HSA has a significantly longer half-life and produces significant tumor targeting due to the introduction of HSA (Burger A M, 2001).

Human serum albumin is an ideal drug carrier due to its stability *in vivo,* safety, non-toxicity, low immunogenicity, biodegradability and tumor- or inflammatorytarged. HSA-based drug long-lasting technology mainly includes the construction of HSA fusion protein, coupling to HSA through covalent chemical bonding, and reversible binding to HSA through non-covalent bonding, and so on. HSA fusion technology is fused with a target protein/polypeptide drug either directly or by a linker. The first FDA-approved HSA fusion protein drug, Albiglutide, is a fusion protein of GLP-1 analogue (8Gly) connecting with HSA in series, with a half-life of up to 5 days, and can be administered once a week. The advantage of HSA fusion links directly to the target protein/polypeptide at the DNA level without additional chemical modification, howver, it needs simple purification and preparation process. It is easier to quality control. However, the disadvantages of HSA fusion proteins have less biological activity *in vitro,* and heterogeneity, easy degradation and low expression levels (Zhao HL, 2008).

In contrast to fusion techniques, coupling HSA to a drug molecule by chemical coupling techniques does not alter the drug molecule structure, especially in the case of proteins and polypeptides, and has less impact on the active structural domains of the drugs. The lysine residue (Lys) is an important functional group for establishing the molecular linkage between HSA and the drug. The current HSA:drug conjugates as a carrier are mostly covalently linked by the drug itself or its derivatives to the amino group of the Lys residue of HSA in the presence of a cross-linking agent. Small molecule drugs bound to HSA by amide bonds using this approach include methotrexate, curcumin and adriamycin etc. (Hoogenboezem and Duvall 2018). The advantage of this coupling approach is that multiple Lys of HSA can be used. However, the lack of selectivity may affect the binding of HSA to FcRn and the consequent pharmacokinetics of HSA (Kuhlmann et al. 2017). Furthermore, this approach may make it difficult to control the number of modifications and sites for each HSA. For example, MTX-HSA is the earliest HSA chemical conjugate in clinical practice, and its biggest drawback is that MTX does not bind to HSA in equal amounts, since there are many Lys residues of HSA, only the average binding ratio of the reaction is 1:1, and the chemical structure of this conjugate is not clear, and its decomposition rate decomposition products are not yet clear (Fiehn et al. 2004).

Cys34 in HSA is another important functional group for molecular linkage between HSA and drugs. The free sulfhydryl group may be specifically coupled to a reactive-to-sulfhydryl group such as maleimide, and the Michael addition reaction between them is highly selective, rapid and under mild conditions. Since Cys34 is located on the surface of HSA , which is far from other ligand binding sites. Furthermore, the Cys34-coupled compounds do not interfere with the affinity and biological activity of HSA with other ligands. For this reason, Cys34 is an ideal site for targeted coupling of drugs in HSA.

The HSA currently used in the market has a potential risk of contamination by viruses and prion because of derived from plasma. More importantly, plasma-derived HSA may have low free sulfhydryl level of HSA since its Cys34 may bind certain heavy metal ions, glutathione or cysteine, whereas recombinant human serum albumin does not have these problems. Characterization of plant-derived recombinant human serum albumin (OsrHSA) shows the same as primary and tertiary structures of HSA. Non-clinical and clinical studies have shown that OsrHSA is safe, well tolerated and efficacy non-interfering to pHSA. It demonstrated that OsrHSA can replace pHSA with advantages of unique, non-plasma-dependent and virus-free. More importantly, Cys34 in OsrHSA is not reduced by glutathione, in that it has a higher content of free sulfhydryl group, which is more beneficial for the preparation of conjugates.

### SUMMARY

In alternative embodiments, provided herein is a drug conjugate of human serum albumin and a therapeutic drug.

In alternative embodiments, provided herein are methods of preparing the drug conjugate of recombinant human serum albumin and a therapeutic drug.

Recombinant human serum albumin has a plurality of modifiable sites, and the free sulfhydryl group on Cys34, as used in drug conjugates as provided herein, is more efficient in the coupling to a therapeutic drug. The coupling of a therapeutic drug to recombinant human serum albumin as provided for in exemplary drug conjugates as provided herein can significantly increase the molecular weight of the coupled drug, an reduce renal clearance and can prolong half-life *in vivo* and prevent from the serum clearance rate, moreover, exemplary drug conjugates as provided herein can be tumor- and inflammation- targeted and reduce the toxic effects of the drug.

In alternative embodiments, provided are human serum albumin conjugates, characterized in that the conjugate has the following structure of:
a recombinant human serum albumin (Cys34) - a heterobifunctional linker - a drug molecule,
wherein the sulfhydryl group at position 34 of the recombinant human serum albumin and the drug molecule are each covalently coupled to the heterobifunctional linker.

**In** alternative embodiments, the recombinant human serum albumin is derived from yeast or rice endosperm cell, optionally recombinant human serum albumin (OsrHSA) is expressed in rice endosperm cells.

Further, the recombinant human serum albumin is a high purity, clinical grade recombinant human serum albumin.

**In** the recombinant human serum albumin conjugate of the present invention, the linker has a heterobifunctional group.

**In** alternative embodiments, the bifunctional group has a reactive-to-sulfhydryl group and a group reactive to one of amino, hydroxyl, carbonyl and carboxyl, wherein the reactive-to-sulfhydryl group is covalently coupled to the 34th cysteine of the recombinant human serum albumin; and the group reactive to amino, hydroxyl, carbonyl, or carboxyl is covalently coupled to the drug molecules.

In the recombinant human serum albumin conjugate of the present invention, the drug molecule is a protein, polypeptide or small molecule compound with a molecular weight of less than 80 kDa and without free sulfhydryl groups.

Further, the drug molecule includes, but is not limited to, lactoferrin, growth hormone, insulin, Teriparatide, Amphotericin B and a derivative thereof.

In a second aspect, the present invention provides a method of preparing the drug conjugate of recombinant human serum albumin and a therapeutic drug, which comprises the following steps of:
(1) covalently coupling a therapeutic drug molecule to one end of a heterobifunctional linker, thereby generating a therapeutic drug-linker conjugate;
(2) removing, or substantially removing, excess uncoupled heterobifunctional linker by desalting or chemical separation, thereby obtaining a preparation comprising a therapeutic drug-linker conjugate having no or substantially no excess uncoupled heterobifunctional linker;
(3) adding a recombinant human serum albumin to the preparation under conditions wherein the recombinant human serum albumin chemically couples to the therapeutic drug-linker conjugate, thereby generating a therapeutic drug-linker-albumin conjugate.

The present invention also discloses the advantages of the recombinant human serum albumin conjugate over human serum albumin in terms of coupling efficiency regarding to the oxidated/reduced status of Cys 34. The advantages are mainly reflected in the fact that the coupling efficiency of recombinant human serum albumin to the drug molecule is increased by approximately 50% compared to human serum albumin derived from plasma (pHSA), which can significantly reduce the cost of the coupled drug. Further analysis reveals that the content of free sulfhydryl groups in the recombinant human serum albumin is approximately three times higher than that of pHSA.

In order to visually demonstrate the method of preparing the drug conjugate according to the present invention and the advantages of the drug conjugate prepared by recombinant human serum albumin over that prepared by pHSA conjugate, the drugs with different molecular weights, such as lactoferrin, growth hormone, insulin, Teriparatide and Amphotericin B are used as target drugs and 6-maleimidohexanoic acid N-hydroxysuccinimide ester (EMCS) is used as a representative linker. The present invention describes in detail the preparation of the recombinant human serum albumin conjugate and compares its coupling efficiency to that of pHSA, showing that the coupling efficiency of the recombinant human serum albumin to the drug is significantly higher than that of pHSA.

The details of one or more exemplary embodiments of the invention are set forth in the accompanying drawings and the description below. Other features, objects, and advantages of the invention will be apparent from the description and drawings, and from the claims.

### DESCRIPTION OF THE DRAWINGS

FIG. 1A illustrates an image of an SDS-PAGE of coupling products of OsrHSA to recombinant human lactoferrin (OsrhLF) and human serum albumin derived from plasma (pHSA) to OsrhLF, respectively, and
FIG. 1B graphically illustrates quantitative analysis of the coupling product bands (** indicates p < 0.01 compared to HSA).
FIG. 2A illustrates an image of an SDS-PAGE of coupling products of OsrHSA to OsrhGH and pHSA to OsrhGH, respectively, and
FIG. 2B graphically illustrates quantitative analysis of the coupling product bands (** indicates p < 0.01 compared to HSA).
FIG. 3A illustrates an image of an SDS-PAGE of coupling products of OsrHSA to Teriparatide and pHSA to Teriparatide, respectively, and
FIG. 3B graphically illustrates quantitative analysis of the coupling product bands (** indicates p < 0.01 compared to HSA).
FIG. 4A illustrates an image of an SDS-PAGE of coupling products of OsrHSA to Amphotericin B and pHSA to Amphotericin B, respectively, and
FIG. 4B graphically illustrates quantitative analysis of the coupling product bands (** indicates p < 0.01 compared to HSA).
FIG. 5 graphically illustrates pharmacokinetic curves of OsrHSA and OsrHSA-EMCS-OsrhGH.
FIG. 6 graphically illustrates an *in vitro* cytological bioactivity assay of rhGH standard, and OsrHSA-ECMS-OsrhGH.
FIG. 7A graphically illustrates data from a purification chromatogram; and
FIG. 7B illustrates an SDS-PAGE of the coupling product OsrHSA-SPDP-Insulin.
FIG. 8 graphically illustrates blood glucose change in diabetic mice injected subcutaneously with OsrHSA-SPDP-Insulin at two different dosages (50 IU/kg and 25 IU/kg), and with saline, and with insulin control.

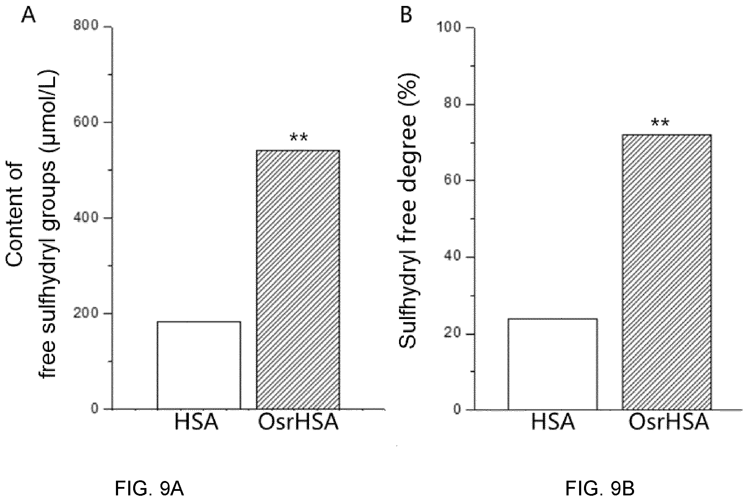

FIG. 9A graphically illustrates the contents of free sulfhydryl groups; and
FIG. 9B graphically illustrates the contents of sulfhydryl free rate (free sulfhydryl molar concentration/protein molar concentration),
in OsrHSA and pHSA at the concentration (50 mg/ml and 752 µmol/L, respecticvely. (** indicates p < 0.01 compared to pHSA).
FIG. 10A graphically illustrates chromatograms and FIG. 10B illustrates SDS-PAGE results of 3 batches from processing validation of OsrHSA-EMCS-Insulin; for FIG. 10B, the SDS-PAGE assay: (01-03 indicate 3 different batches, respectively).
FIG. 11A-B illustrates glucose tolerance test in rats: FIG. 11A schematically illustrates a diagram of the timepoints of blood sampling; FIG. 11B graphically illlustrates blood glucose concentration curves at different timepoints.
FIG. 12A graphically illustrates blood glucose; and FIG. 12B graphically illustrates blood drug concentration curves of rats given a single dose under non-fasting conditions.

### DETAILED DESCRIPTION

In alternative embodiments, provided are drug-linker-albumin conjugates comprising: (a) a recombinant human serum albumin; (b) a therapeutic drug, and (c) a heterobifunctional linker,
wherein the drug conjugate has a structure comprising: the recombinant human serum albumin is conjugated or linked to the therapeutic drug molecule by the heterobifunctional linker,
wherein a sulfhydryl group at position 34 of the recombinant human serum albumin is covalently coupled to the therapeutic drug by the heterobifunctional linker.

In alternative embodiments of drug-linker-albumin conjugates as provided herein:
- the recombinant human serum albumin is a recombinantly expressed *in vitro.;*
- the recombinant human serum albumin is a plant-derived or plant expressed recombinant human serum albumin;
- the plant-derived recombinant human serum albumin is a recombinant human serum albumin expressed in rice seeds, yeast or a rice endosperm cell;
- the heterobifunctional linker has bifunctional groups which comprise a reactive-to-sulfhydryl group and a group reactive to one of amino, hydroxyl, carbonyl and carboxyl;
- the reactive-to-sulfhydryl group of the heterobifunctional linker is covalently coupled to the cysteine at position 34 of the recombinant human serum albumin, and the group reactive to one of amino, hydroxyl, carbonyl and carboxyl of the linker is covalently coupled to the therapeutic drug molecule;
- the therapeutic drug molecule is a protein, polypeptide or small molecule compound with a molecular weight of less than about 80 kDa and without a free sulfhydryl group;
- the therapeutic drug molecule is selected from the group consisting of: lactoferrin, a growth hormone, insulin, a parathyroid hormone (PTH), teriparatide (or FORTEO^{™}), amphotericin B and a chemical drug derivative thereof; and/or
- the heterobifunctional linker comprises a 6-maleimidohexanoic acid N-hydroxysuccinimide ester.

In alternative embodiments, provided are methods of preparing the drug-linker-albumin conjugate as provided herein, comprising the following steps of:
(1) covalently coupling a therapeutic drug molecule to one end of a heterobifunctional linker, thereby generating a therapeutic drug-linker conjugate;
(2) removing, or substantially removing, excess uncoupled heterobifunctional linker by desalting or chemical separation, thereby obtaining a preparation comprising a therapeutic drug-linker conjugate having no or substantially no excess uncoupled heterobifunctional linker;
(3) adding a recombinant human serum albumin to the preparation under conditions wherein the recombinant human serum albumin chemically couples to the therapeutic drug-linker conjugate, thereby generating a therapeutic drug-linker-albumin conjugate.

In alternative embodiments of methods as provided herein:
- the heterobifunctional linker comprises 6-maleimidohexanoic acid N-hydroxysuccinimide ester; Maleimidoacetic Acid N-Hydroxysuccinimide Ester; Sulfo-SMCC, is a sulfhydryl- and amine-reactive crosslinker; 5-Azido-2-nitrobenzoic acid N-hydroxysuccinimide ester; Benzophenone-4-isothiocyanate; N-Succinimidyl Iodoacetate; N-Hydroxysuccinimidyl-4-azidobenzoate; N-Succinimidyl-p-Formylbenzoate; 3-(2-Pyridyldithio)propionic Acid N-Succinimidyl Ester; 4-(4-Maleimidophenyl)butyric acid N-hydroxysuccinimide ester; 4-Maleimidobutyric Acid N-Succinimidyl Ester; 4-[3-(Trifluoromethyl)diazirin-3-yl]benzoic Acid N-Hydroxysuccinimide Ester; 4-(N-Succinimidylcarboxy)benzophenone; N-Succinimidyl 4-Azido-2,3,5,6-tetrafluorobenzoate; Succinimidyl 6-[3-(2-Pyridyldithio)propionamido]hexanoate; 4-(Maleimidomethyl)cyclohexane-1-carboxyl-hydrazide, Trifluoroacetic Acid; or, 6-Maleimidocaproic Acid Sulfo-N-Succinimidyl Ester, or

| | |
|---|---|
| 6-maleimidohexanoic acid N-hydroxysuccinimide ester | 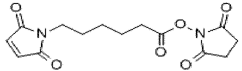 |
| Maleimidoacetic Acid N-Hydroxy succinimide Ester | 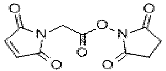 |
| sulfosuccinimidyl 4-(N-maleimidomethyl) cyclohexane-1-carboxylate | 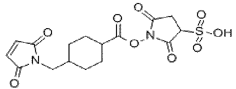 |
| 5-Azido-2-nitrobenzoic acid N-hydroxysuccinimide ester | 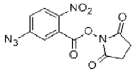 |
| Benzophenone-4-isothiocyanate | 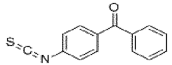 |
| N-Succinimidyl Iodoacetate | 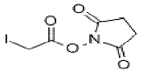 |
| N-Hydroxysuccinimidyl-4-azidobenzoate | 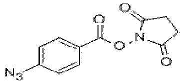 |
| N-Succinimidyl-p-Formylbenzoate | 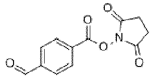 |
| 3-(2-Pyridyldithio)propionic Acid N-Succinimidyl Ester | 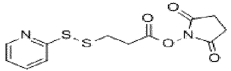 |
| 4-(4-Maleimidophenyl)butyric acid N-hydroxysuccinimide ester | 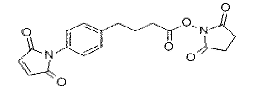 |
| 4-Maleimidobutyric Acid N-Succinimidyl Ester | 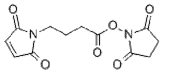 |
| 4-[3-(Trifluoromethyl)diazirin-3-yl]benzoic Acid N-Hydroxysuccinimide Ester | 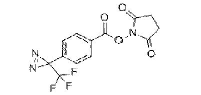 |
| 4-(N-Succinimidylcarboxy)benzophenone | 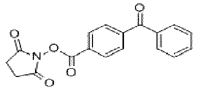 |
| N-Succinimidyl 4-Azido-2,3,5,6-tetrafluorobenzoate | 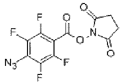 |
| Succinimidyl 6-[3-(2-Pyridyldithio) propionamido]hexanoate | 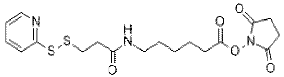 |
| 4-(Maleimidomethyl)cyclohexane - 1-carboxyl-hydrazide Trifluoroacetic Acid | 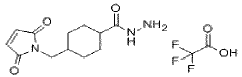 |
| 6-Maleimidocaproic Acid Sulfo-N-Succinimidyl Ester | 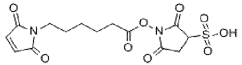 |
| 2,5-dioxopyrrolidin-1-yl 6-(3-bromo-2,5-dioxo-2,5 -dihydro-1H-pyrrol-1-yl) hexanoate | 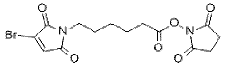 |
| 2,5-dioxopyrrolidin-1-yl 6-acrylamidohexanoate | 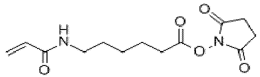 |
| (Z)-2,5-dioxopyrrolidin-1-yl 6-(3-ethylidene-2,5-dioxopyrrolidin-1-yl)hexanoate | 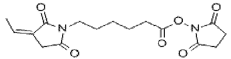 |
| 2,5-dioxopyrrolidin-1-yl 6-(3-methyl-2,5-dioxo-2,5-dihydro-1H-pyrrol-1-yl)hexanoate | 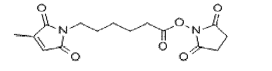 |
| 2,5-dioxopyrrolidin-1-yl 8-(3-(2,5-dioxo-2,5-dihydro-1H-pyrrol-1-yl) propanamido) octanoate | 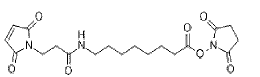 |
| 4-((S)-2-((S)-2-(6-(2,5-dioxo-2,5-dihydro-1H-pyrrol-1-yl)hexanamido)-3-methylbutanamido) -5-ureidopentanamido)benzyl (4-nitrophenyl) carbonate | 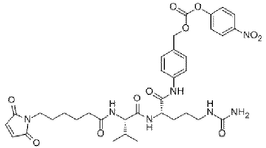 |

; and/or
- the therapeutic drug reacts with the heterobifunctional linker at a molecular molar ratio of 1:5 in step (1), and in step (2) the recombinant human serum albumin reacts with the therapeutic drug-linker conjugate at a molecular molar ratio of 1:(0.2-1).

### Reaction Conditions

In alternative embodiments, conditions for practicing methods as provided herein comprise:
In alternative embodiments, for polypeptides or protein drugs, the drugs are were dissolved in an appropriate buffer, optionally deionized water, distilled water, or PBS, and then the heterobifunctional linker dissolved in a solvent (for example, DMSO, DMF, or equivalent) was slowly added dropwise at a molecule molar ratio of drugs to linker of about 1: 1 to 1: 10, and stirred at room temperature (RT) for at least 15 min.

In alternative embodiments, for chemical drugs, the drugs (1 eq. (equivalent)) were dissolved in appropriate solvent (DMF, DMSO, etc.), then base (2 eq., DIEA, TEA) was added and the mixture was stirred at appropriate temperature (15°C at approximately 60°C) for 10 min. Then the linker (1 eq. approximately 1.2 eq.) was added to the mixture. Stirring was allowed to continue for an additional 2 h approximately 16 h. TLC confirmed the reaction was completed.

In alternative embodiments, methods comprise removing, or substantially removing, excess uncoupled heterobifunctional linker by desalting or chemical separation, and exemplary protocols and conditions comprise:
In alternative embodiments, for polypeptides or protein drugs, due to the low molecular weight of the linker, the excess uncoupled linkers can be removed by ultrafiltration, dialysis, gel filtration, and other methods. For ultrafiltration or dialysis, the molecular weight cut-off of the membrane should be close to 1000 Da. Under this condition, the solvent and linker present in the aqueous phase of the reaction mixture pass through the membrane. Desalting Columns can also be used in buffer exchange and removal of low-molecular weight compounds.

In alternative embodiments, for chemical drugs, the solvent was removed under reduced pressure to give crude product. The crude product was purified by prep-TLC (petroleum ether: ethyl acetate=3:1 approximately dichloromethane: methanol=10: 1) or flash (acetonitrile: water, 1% TFA, flow rate: 20 approximately 100 ml/min, elution gradient was between about 20% to approximately 80%). The solvent was removed with appropriate method to give the desired product.

### Products of manufacture and Kits

Provided are products of manufacture and kits for practicing methods as provided herein; and optionally, products of manufacture and kits can further comprise instructions for practicing methods as provided herein.

Any of the above aspects and embodiments can be combined with any other aspect or embodiment as disclosed here in the Summary, Figures and/or Detailed Description sections.As used in this specification and the claims, the singular forms "a," "an" and "the" include plural referents unless the context clearly dictates otherwise. Unless specifically stated or obvious from context, as used herein, the term "or" is understood to be inclusive and covers both "or" and "and".Unless specifically stated or obvious from context, as used herein, the term "about" is understood as within a range of normal tolerance in the art, for example within 2 standard deviations of the mean. About (use of the term "about") can be understood as within 20%, 19%, 18%, 17%, 16%, 15%, 14%, 13%, 12% 11%, 10%, 9%, 8%, 7%, 6%, 5%, 4%, 3%, 2%, 1%, 0.5%, 0.1%, 0.05%, or 0.01% of the stated value. Unless otherwise clear from the context, all numerical values provided herein are modified by the term "about."Unless specifically stated or obvious from context, as used herein, the terms "substantially all", "substantially most of", "substantially all of" or "majority of" encompass at least about 75%, 80%, 85%, 90%, 91%, 92%, 93%, 94%, 95%, 96%, 97%, 98%, 99% or 99.5%, or more of a referenced amount of a composition.

The invention will be further described with reference to the examples described herein; however, it is to be understood that the invention is not limited to such examples. The contents of the present invention are further illustrated by the following specific examples. The examples are merely provided to illustrate the present invention and do not in any way limit the rest of what is disclosed by the invention.

### EXAMPLES

### Materials and sources:

Human serum albumin derived from plasma (pHSA): commercially available from Grifols, lot No. A3AFE01332;
Recombinant human serum albumin (OsrHSA) derived from rice seeds: prepared according to the patents CN100540667C and CN103880947A;
Recombinant human lactoferrin (OsrhLF, molecular weight 78.6 kDa): prepared according to the patent application publication WO 2020/088207 A1;
Recombinant human growth hormone (OsrhGH, molecular weight 22 kDa): prepared according to patent CN111057138A;
Teriparatide (molecular weight 4252Da): commercially available from Thinheal Pharmaceuticals Co., Ltd.;
Recombinant human insulin (Insulin, molecular weight 5.8 kDa): commercially available from Hefei Tianmai Biotechnology Development Co., Ltd.
All other materials were purchased commercially.

### Example 1: Preparation of exemplary coupling products with OsrHSA or pHSA to recombinant human lactoferrin

Recombinant human lactoferrin (OsrhLF) with a molecular OsrhLF weight 78.6 kDa (prepared according to the patent WO2020088207A1) was dissolved in phosphate buffer solution (PBS), and then EMCS solution (100 mmol/L) was slowly added dropwise at a molecule molar ratio of OsrhLF to EMCS of 1:5, and stirred at room temperature for 30 min. The reaction was terminated by adding Glycine solution (1 mol/L), which was 5 folds of the molar concentration of EMCS.

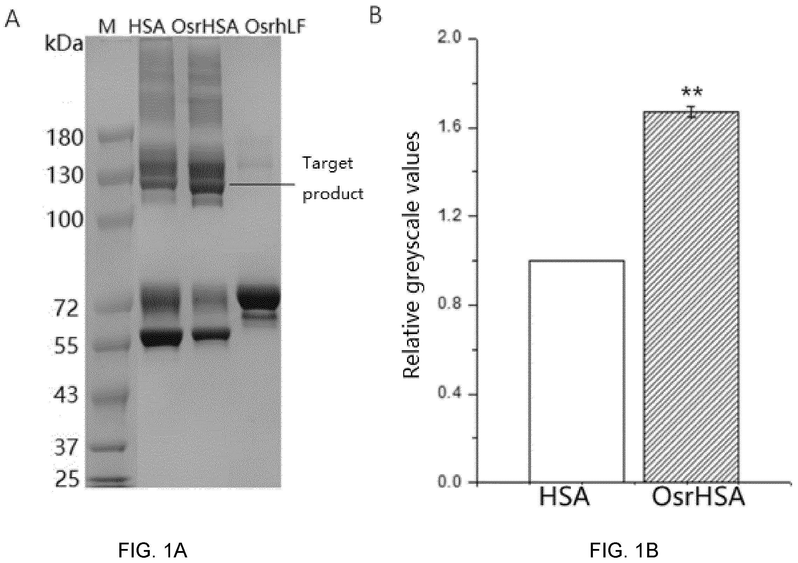

After the reaction, excess EMCS was removed using a Bestdex G-25 M desalting column. OsrhLF-EMCS after desalination was divided into 2 parts and added with OsrHSA (C001202010002, 20%) and pHSA (Grifols, lot No. A3AFE01332, 20%), respectively, at a molecular molar ratio of 1:0.5; and reacted at room temperature for 1h. An appropriate amount of each reaction solution was used for the analysis of the coupling efficiency of OsrHSA or pHSA to OsrhLF by 4-20% sodium dodecyl sulfate-polyacrylamide gel electrophoresis (SDS-PAGE). As shown in Figure 1, the results indicated that the bands of the coupling product (target product) in the OsrHSA coupling reaction were significantly more than those in the pHSA coupling reaction. The content of the coupled product with OsrHSA was approximately 1.67-folds higher than that in the pHSA, as calculated by Image J greyscale scanning (Figure 1).

### Example 2: Preparation of exemplary coupling products with OsrHSA or pHSA to recombinant human growth hormone

Recombinant human growth hormone (OsrhLF) with molecular weight 22 kDa was prepared according to the patent CN111057138A, and was dissolved in phosphate buffer solution (PBS). And then EMCS solution (100 mmol/L) was slowly added dropwise at a molecule molar ratio of OsrhGH to EMCS of 1:5 and stirred at room temperature for 30 min. The reaction was terminated by adding Glycine solution (1 mol/L), which was 5 folds the molar concentration of EMCS.

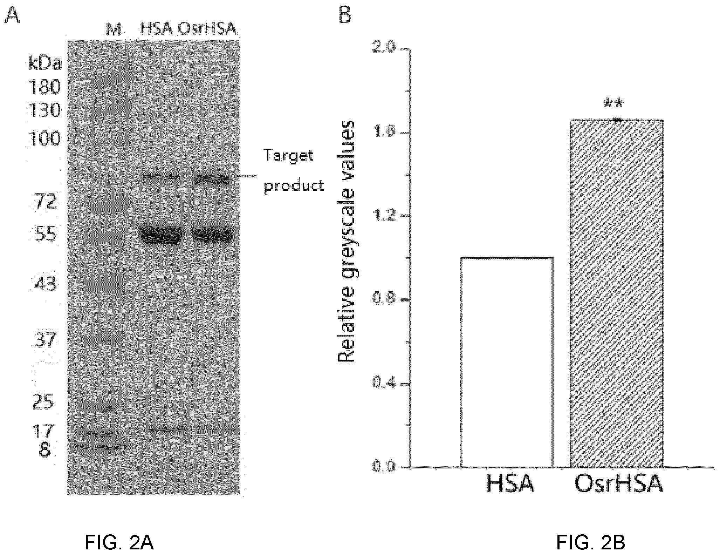

After the reaction, excess EMCS was removed using a Bestdex G-25 M desalting column. OsrhGH-EMCS after desalination was divided into two parts and added with OsrHSA or HSA injection, respectively, with a molecular molar ratio of 1:1. The reaction was conducted at room temperature for 1h. Then the coupling efficiency of OsrHSA or pHSA to OsrhGH was analyzed by 4-20% SDS-PAGE. As shwon in Figure 2, the results indicated that coupled products with OsrHSA were significantly more than those in the products coupled with pHSA. The content of coupled product in the OsrHSA reaction solution was approximately 1.66 times higher than that of with pHSA, as calculated by Image J greyscale scanning (Figure 2).

### Example 3: Preparation of exemplary coupling products with OsrHSA or pHSA to Teriparatide

22 mg of teriparatide (Thinheal Pharmaceuticals, molecular weight 4252 Da) was dissolved in ultrapure water (4.25mg/ml). EMCS stock solution (100mmol/L) was added at a molar ratio of Teriparatide to EMCS of 1:2, and then stirred at room temperature for 30min. The reaction was terminated by adding 1mol/L Glycine.

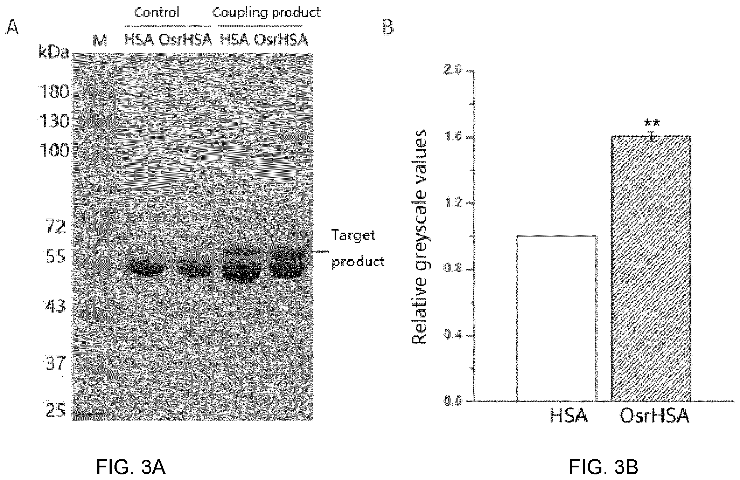

After the reaction, excess EMCS was removed using a Bestdex G-25 M desalting column. Teriparatide-EMCS after desalination was divided into two parts, and added with OsrHSA and pHSA, respectively, with a molecular molar ratio of 1:1. The reactions were conducted at room temperature for 1h. Then the coupling efficiency of OsrHSA or pHSA to Teriparatide was analyzed by 4-20% SDS-PAGE. As shown in Figure 3, the resulst indicated that the content of the coupled product with OsrHSA was significantly higher than that with pHSA. The content of coupled product with OsrHSA was approximately 1.61 times higher than that with pHSA, as calculated by Image J greyscale scanning (Figure 3).

### Example 4: Preparation of exemplary coupling products with OsrHSA or pHSA to Amphotericin B

100 mg of Amphotericin B (AmB, MW 924.09) was dissolved in 5 ml of N,N-dimethylformamide at 25°C under dark, then N,N-diisopropylethylamine (37 µl, 0.216 mmol), hydroxybenzotriazole (5 mg, 32.5 µmol) and maleimidocaproyl-L-valine-L-citrulline-p-aminobenzyl alcohol p-nitrophenyl carbonate (Mc-Val-Cit-PABC-PNP, 96 mg, 0.13 mmol) were stepwisely added with stirring, respectively. The reaction solution was stirred at 25°C for 5 h under dark. Thin layer chromatography (TLC, dichloromethane: methanol = 5:1) and LC-MC showed that Amphotericin B was completely reacted. The coulped products was concentrated, and then 10 ml of methanol was added to dissolve the concentrated product. After then, 30 ml of methyl tert-butyl ether was slowly added dropwise to the reaction flask with stirring. The suspension was stirred at room temperature for 10 min, the filter cake was collected by filtration through negative pressure. The coupled products were dried with a yellow solid product, AmB-Mc-Val-Cit-PAB-PNP (AmB-MVCPP, MW: 1521.76).

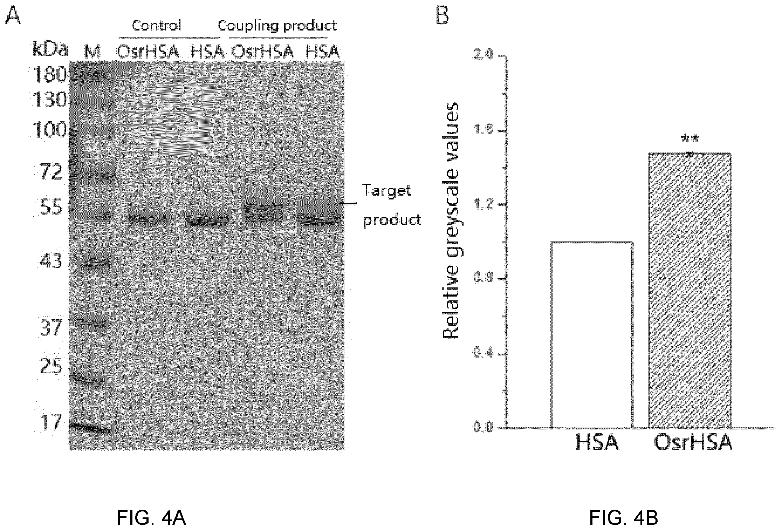

AmB-MVCPP was dissolved in DMSO (10 mmol/L), divided into two parts, and then added OsrHSA and HSA at a molecular molar ratio of 1:0.2, respectively. The reaction was carried out at room temperature overnight. Then the coupling efficiency of OsrHSA or pHSA to AmB was analyzed by 8% SDS-PAGE. As shown in Figure 4, the results indicated that the coupled product with OsrHSA was significantly higher than that with pHSA. The content of coupled product with OsrHSA was approximately 1.47 times higher than that with p HSA, as calculated by Image J greyscale scanning (Figure 4).

### Example 5: Pharmacokinetic study of exemplary OsrHSA-EMCS-OsrhGH in mice

To investigate whether the half-life of OsrHSA-EMCS-OsrhGH was significantly longer than that of OsrhGH, the OsrHSA-EMCS-OsrhGH prepared in Example 2 further was purified by Phenyl HP and Nano 50Q to remove unreacted OsrHSA and OsrhGH. The final product was injected subcutaneously into Kunming mice with a dose of 5 mg/kg (based on OsrhGH). Blood was then taken from the orbital vein of the mice at different timepoints after administration. The OsrhGH content in serum was determined using the Human Growth Hormone (GH) DuoSet ELISA (DY1067) kit. The pharmacokinetic parameters of OsrhGH were calculated based on the OsrhGH content in serum at different timepoints.

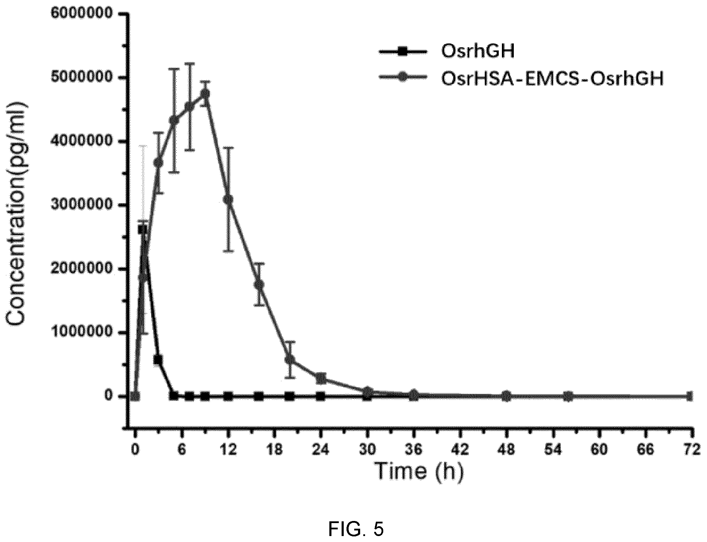

As shown in Table 1, the half-life (t1/2) of OsrhGH in mice was 0.47h, while the half-life of OsrHSA-EMCS-OsrhGH was 7.39h, approximately 16 times longer than that of OsrhGH, indicating that the half-life of OsrhGH coupled to OsrHSA was significantly prolonged. The peak time (Tmax) of OsrhGH was 1h, while the peak time of OsrHSA-EMCS-OsrhGH was 9h, namely, the action duration of OsrHSA-EMCS-OsrhGH was significantly longer than that of OsrhGH. The peak concentration (Cmax) of OsrHSA-EMCS-OsrhGH was approximately twice that of OsrhGH, while the area under the drug-time curve (AUClast) of OsrHSA-OsrhGH was approximately 12 times that of OsrhGH. In summary, OsrhGH coupled with OsrHSA had a significantly longer half-life, peak time, peak concentration and AUClast than that of OsrhGH alone (Figure 5).

**Table 1 Pharmacokinetic parameters of OsrHSA-EMCS-OsrhGH**

| Treatments | T1/2 (h) | Tmax (h) | Cmax (ng/ml) | AUClast (ng/ml) |
|---|---|---|---|---|
| OsrhGH | 0.47 | 1 | 2616.50 | 5057.63 |
| OsrHSA-EMCS-OsrhGH | 7.39 | 9 | 4750.82 | 62033.48 |

### Example 6: In vitro activity assay of OsrHSA-EMCS-OsrhGH

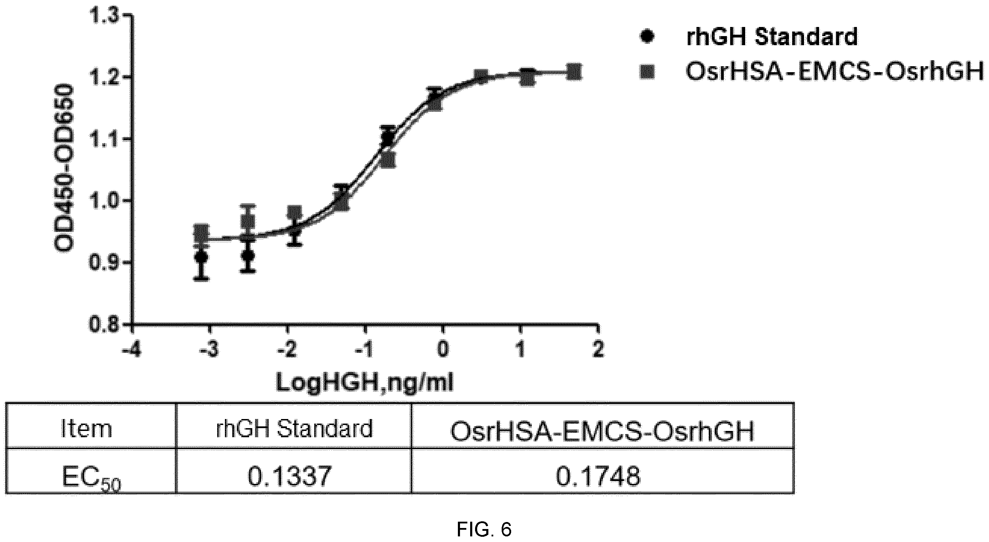

In order to verify whether OsrHSA-EMCS-OsrhGH has biological activity, OsrHSA-EMCS-OsrhGH prepared in Example 2 was subjected to carried out biological activity assay *in vitro* using mouse lymphoma NB2-11 cell. The results are shown in Figure 6. OsrHSA-EMCS-OsrhGH can significantly promote the growth and proliferation of mouse lymphoma NB2-11 cells. The number of the cells gradually increased with the increasing concentration of OsrHSA-EMCS-OsrhGH, showed well relationship of the effects-concentration. Compared to the rhGH (positive control), OsrHSA-EMCS-OsrhGH presented the same promoting effect of mouse lymphoma NB2-11 cell as rhGH, indication coupled product has a similar biological activity.

### Example 7: Preparation of exemplary coupled products with OsrHSA to recombinant human insulin (OsrHSA-SPDP-Insulin)

### 7.1 Coupling reaction of recombinant human insulin (Insulin) to SPDP

42 ml of Insulin solution (0.4 mmol/L dissolved in coupling solution) was slowly added dropwise with 8 ml of N-succinimidyl 3-(2-pyridinedithio)propionate (SPDP) solution (100 mmol/L, dissolved in DMSO) with a molecular molar ratio of Insulin to SPDP of 1:5, then stirred at room temperature for 1 h. And then the reaction was terminated by adding Glycine solution, which was 5-folds of the molar amount of SPDP.

### 7.2 Coupling of Insulin-SPDP to OsrHSA

After the completion the reaction, excess SPDP and DMSO were removed with a Bestdex G-25 M desalting column. The Insulin-SPDP after desalting was added with 3.88 ml of OsrHSA solution (3 mmol/L) at a molecular molar ratio of Insulin-SPDP to OsrHSA of 1:1, then mixed thoroughly and left to react at 4°C for 18h.

### 7.3 Purification of the coupling product OsrHSA-SPDP-Insulin

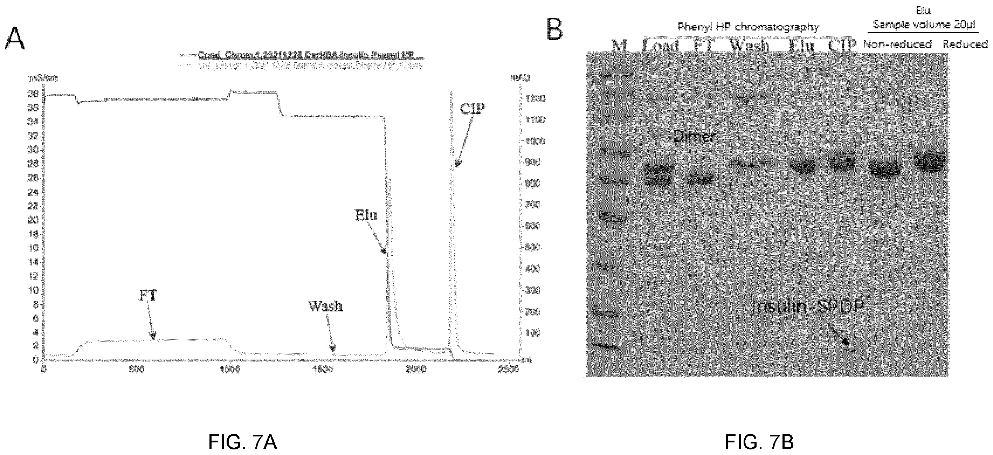

First, the conductance of the coupled product solution was adjusted with 3M ammonium sulfate to consistence to the equilibrium buffer (10 mM PB, 0.2 M ammonium sulfate, pH 6.5). And then the equilibrium buffer was used to dilute the coupled product to final concentration approximately 1 mg/ml (based on OsrHSA). The coupled product was loaded onto a column filled with 176 ml of Phenyl Bestrose HP chromatography resin with a column height of 25 cm at a flow rate of 15 ml/min. After the sample loading, the chromatographic column was equilibrated again with the equilibration buffer until the UV was the same as the baseline. The dimer of the coupled product was removed using a washing buffer (10 mM PB, 0.18M ammonium sulfate, pH 6.5). Finally the coupled product was eluted using an elution buffer (10 mM PB, pH 7.2). The eluate was concentrated and ultrfiltrated using a 50 kDa ultrafiltration membrane pack. The stock solution of OsrHSA-SPDP-Insulin conjugate was obtained. The purity of the final OsrHSA-SPDP-Insulin as shown in chromatogram (A) and SDS -PAGE assay results (B) of the OsrHSA-SPDP-Insulin coupling product are shown in Figure 7.

### Example 8 The long-lasting efficacy of OsrHSA-SPDP-Insulin for the hyperglycaemia in a diabetic mouse model

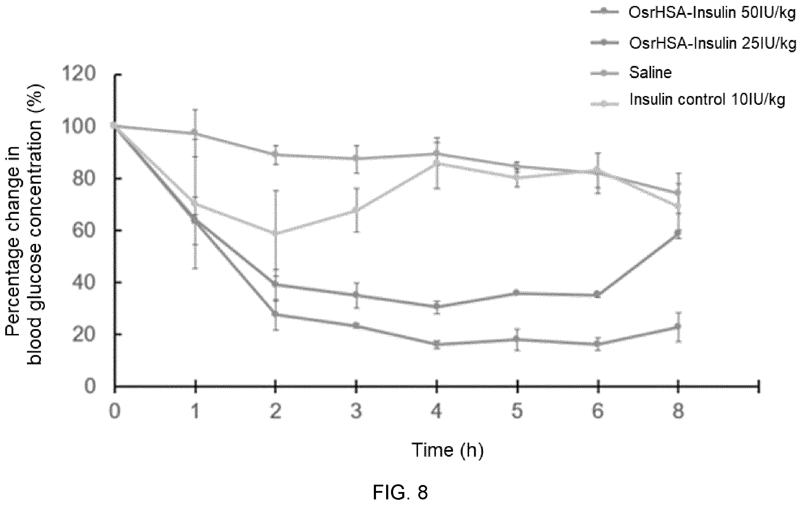

To demonstrate the long-lasting efficacy of OsrHSA-SPDP-Insulin for hyperglycaemia, a male BSK-DB diabetic mouse model was used for the study. OsrHSA-SPDP-Insulin was prepared as described in Example 7. After the mice were fasted overnight without water deprivation, five mice each group were received single dose by subcutaneous infusion of OsrHSA-SPDP-Insulin with doses of of 25 IU/kg and 50 IU/kg, positive control Insulin with a dose of 10 IU/kg, and negative control (an equal volume of saline. Blood samples were collected from the tail vein of the mice at before dosing and 8h after the dosing. The blood glucose level was detected using a Roche blood glucose meter and testing strips. The blood glucose change curves were plotted according to the change value (Tₙ/T₀) in blood glucose value (Tₙ) relative to the initial blood glucose value (T₀) at different timepoints. As shown in Figure 8, the blood glucose level in the insulin control group dramatically dropped 2h after administration and returned to the level of the saline group at about 4h, while OsrHSA-SPDP-Insulin (OsrHSA-Insulin) showed a more significant pronounced hypoglycaemic effect i.e., greater than (>) 8 hours (h), presenting a significantly longer duration of action. These results demonstrated that OsrHSA-Insulin can significantly prolong the duration of the coupled drugs.

### Example 9: Determination of the content of free sulfhydryl groups in OsrHSA and HSA

In order to elucidate the mechanism by which the coupling efficiency of OsrHSA is higher than that of pHSA, the content of free sulfhydryl groups was determined by the DTNB method. OsrHSA or pHSA was diluted to 50 mg/ml (752 µmol/L) using a reaction buffer (100 mmol/L sodium phosphate, 1 mmol/L EDTA, pH 8.0) for in use. In a 5 ml reaction tube, 2.5 ml of reaction buffer and 50 µl of DTNB reaction solution (4 mg/ml) were added first, and then added 250 µl of cysteine standard solution (0.25 approximately 1.5 mmol/L) or the sample to be tested, respectively. The reaction was carried out at room temperature for 15 min. And then the OD₄₁₂ values were measured using a spectrophotometer. A standard curve was plotted based on the absorbance of the cysteine standard solution, and then the sulfhydryl content was calculated. The results indicated that the free sulfhydryl content in OsrHSA of 541 µmol/L of 752 µmol/L was sulfhydryl free, accounting for 72% sulfhydryle free, while the free sulfhydryl content in pHSA was 183 µmol/L out of 752 µmol/L, accoutinmg for 24% only. The content of free sulfhydryl groups in OsrHSA is three folds higher than that of pHSA.

### Example 10 Preparation of exemplary OsrHSA-EMCS-Insulin at high loading capacity

400 mg of Insulin dry powder (purchased from Eastern Antibiotics, Calt No. GMP-045) was dissolved in 20 ml of 4 mmol/L dilute hydrochloric acid (pH 2.0), and slowly adjusted to pH 7.2 (pH should not exceed 8.0) using 0.5 mol/L NaOH. And then supplemented with a coupling buffer (20 mmol/LPB, 2 mmol/LEDTA, pH 7.2) to 100 ml. After then 3.43 ml of EMCS solution (31 mg/ml) was slowly added dropwise with stirring at a mass ratio of Insulin to EMCS of 1:0.266 (molar ratio 1:5), and stirred at 25°C for 30 min. The reaction was terminated by adding Glycine, which was 5 times the molar amount of EMCS.

Excess EMCS was removed using a G-25 desalting column (column volume 490 ml, flow rate 25-40 ml/min) . The desalted Insulin-EMCS was diluted to approximately 1600 ml using the coupling buffer. Then 50.9 ml of OsrHSA (153 mg/ml) was added with a mass ratio of Insulin-EMCS:OsrHSA of 1:22.9 (molar ratio 1:2), the final concentration of OsrHSA in the system was 5 mg/ml). The reaction was carried out at 25°C for 50 min. The reaction was terminated by adding Cys, which was 5 times the molar amount of OsrHSA.

The coupling product was diluted to approximately 1 mg/ml (based on OsrHSA) and purified using a Phenyl HP chromatography column. The eluatant was concentrated and ultrafiltrated using a 50 kDa ultrafiltration membrane pack, then at least two times the volume of 10 mmol/LPB (pH 7.2) was added, concentrated for dialysis and repeated 7 times, the resulting of OsrHSA-EMCS-Insulin stock solution was obtained.

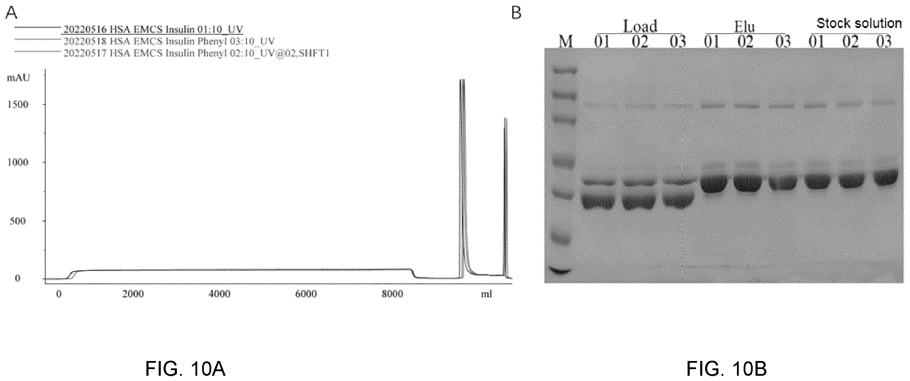

The coefficient of variation of the chromatograms and SDS-PAGE indicated that the yield from the three validated batches were less than 10%, showing that it is excellent consistence and the monomer of OsrHSA-EMCS-Insulin purity are 94.94% (Table 1, Table 2 and Figure 10).

**Table 1 The recovery rate during the purification processes**

| Batches | | | | Mean value | CV (%) |
|---|---|---|---|---|---|
| | 20220516 | 20220517 | 20220518 | | |
| The recovery rate of Insulin during the process (%) | 30.82 | 29.71 | 28.87 | 29.80 | 3.29 |
| The recovery rate of Insulin after the ultrafiltration (%) | 88.37 | 104.54 | 94.82 | 95.83 | 8.49 |
| Total recovery rate of insulin (%) | 27.24 | 31.06 | 27.37 | 28.56 | 7.60 |

**Table 2**

| The purity of OsrHSA-EMCS-Insulin form 3 batches resulting product | | | | | | |
|---|---|---|---|---|---|---|
| Batches | 20220516 | 20220517 | 20220518 | Mean value | Standard deviation | CV (%) |
| Polymers (%) | 0.2101 | 0.1884 | 0.1642 | 0.19 | 0.02 | 12.24 |
| OsrHSA dimer (%) | 5.407 | 5.3283 | 5.2465 | 5.33 | 0.08 | 1.51 |
| Coupling product (%) | 94.3829 | 94.4833 | 94.5893 | 94.49 | 0.10 | 0.11 |

### Example 11 Intraperitoneal glucose tolerance test (IPGTT) of exemplary OsrHSA-EMCS-Insulin

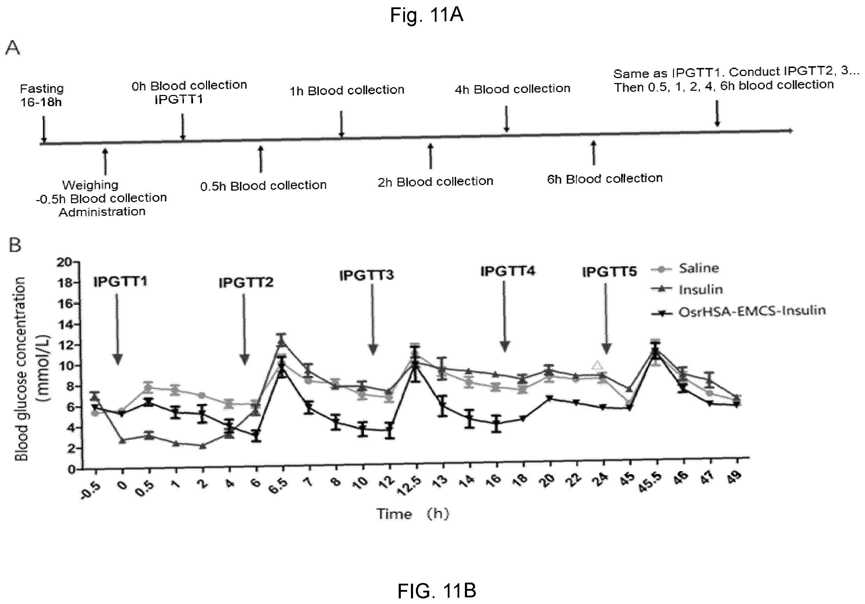

An intraperitoneal glucose tolerance tests (IPGTT) in rat was performed for OsrHSA-EMCS-Insulin that was prepared according to Example 10. The test was divided into three groups, the control and two experimental groups with 6 SD rats for each group. The experimental group was administered once by subcutaneous injection at a single dose of 125 nmol/kg and the control group was given the same volume of placebo saline. 16 h before the glucose tolerance test, the rats were fasted without water deprivation. Blood sample was collected from the orbital plexus of rats at -0.5h (baseline fasting blood glucose concentration before administration), 0h (blood glucose concentration before intraperitoneal injection of 20 g/kg glucose at 0.5h after administration), 0.5h, 1h, 2h, 4h and 6h. The blood glucose level were measured at 0.5h, 1h, 2h, 4h and 6h after glucose dosing. The results showed that the efficacy of OsrHSA-EMCS-Insulin could last up to 45-49h compared with the Insulin group and the negative control group, demonstrating that OsrHSA-EMCS-Insulin has a longer efficacy (Figure 11). After 5 rounds of IPGTT, the blood glucose concentration of OsrHSA-EMCS-Insulin was basically the same as that of the control group, indicating that the duration of action of OsrHSA-EMCS-Insulin in rats was about 24h.

### Example 12: Pharmacodynamic and pharmacokinetic studies of exemplary OsrHSA-EMCS-Insulin in SD rats

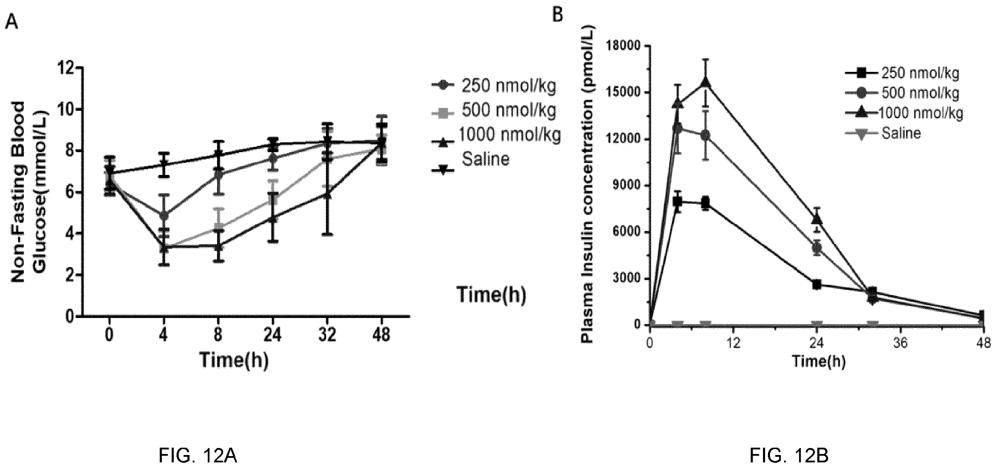

In order to demonstrate the longevity and efficacy of OsrHSA-EMCS-Insulin, SD rats were selected as the study subjects for testing. Seven weeks old SD male rats with 200g-250g body weigh were randomly grouped as non-fasting conditions. OsrHSA-EMCS-Insulin and an equal volume of saline (negative control) were administered once by subcutaneous injection with different doses. Then blood was taken from the orbital plexus of the rats at 4h, 8h, 24h, 32h and 48h after administration and treated at 37°C for 30 min, and then centrifuged at 4000 rpm for 15 min. The supernatant was stored at -80°C for use. The blood glucose and insulin levels were measured using blood glucose test strips (Roche) and Insulin ELISA kit (R&D, DY8056-05). As shown in in Figure 12, OsrHSA-EMCS-Insulin presents a significant dose effect relationship, i.e., the higher dosage corresponding to the better and longer lasting efficacy to the hyperglycemia. At the highest dose of 1000 nmol/kg, efficacy in hyperglycemia SD rats could last for 32-48h. The results showed that the concentration of Insulin in blood was positively correlated with the administered drug dosage in the period of 0-32 h. The blood insulin level in three groups inclined to the background level. It is consistence of the change of blood insulin level with blood glucose concentration.

Data provided herein demonstrates the obvious advantages of the exemplary recombinant human serum albumin over pHSA in terms of more free sulfhydryl-based coupling sites and the preparation method for coupling therapeutic drug.

However, embodiments as provided herein, or the invention, are not limited to the detailed methods described above, i.e., it does not mean that embodiments as provided herein, or the present invention, must rely on the detailed preparation methods described above in order to be implemented. It should be clear to those skilled in the art that any improvements to embodiments as provided herein, or the present invention, substitutions of the drugs coupled to OsrHSA in the present invention, etc., fall within the scope of protection and disclosure of the present invention.

A number of embodiments of the invention have been described. Nevertheless, it can be understood that various modifications may be made without departing from the spirit and scope of the invention. Accordingly, other embodiments are within the scope of the following claims.

## Claims

1. A drug-linker-albumin conjugate comprising:
(a) a recombinant human serum albumin;
(b) a therapeutic drug, and
(c) a heterobifunctional linker,
wherein the drug conjugate has a structure comprising: the recombinant human serum albumin is conjugated or linked to the therapeutic drug molecule by the heterobifunctional linker,
wherein a sulfhydryl group at position 34 of the recombinant human serum albumin is covalently coupled to the therapeutic drug by the heterobifunctional linker.

2. The drug-linker-albumin of claim 1, wherein the recombinant human serum albumin is a recombinantly expressed *in vitro.*

3. The drug-linker-albumin of claim 1, wherein the recombinant human serum albumin is a plant-derived or plant expressed recombinant human serum albumin.

4. The drug-linker-albumin of claim 3, wherein the plant-derived recombinant human serum albumin is a recombinant human serum albumin expressed in rice seeds, yeast or a rice endosperm cell.

5. The drug-linker-albumin of claim 1, wherein the heterobifunctional linker has bifunctional groups which comprise a reactive-to-sulfhydryl group and a group reactive to one of amino, hydroxyl, carbonyl and carboxyl.

6. The drug-linker-albumin of claim 5, wherein the reactive-to-sulfhydryl group of the heterobifunctional linker is covalently coupled to the cysteine at position 34 of the recombinant human serum albumin, and the group reactive to one of amino, hydroxyl, carbonyl and carboxyl of the linker is covalently coupled to the therapeutic drug molecule.

7. The drug-linker-albumin of claim 1, wherein the therapeutic drug molecule is a protein, polypeptide or small molecule compound with a molecular weight of less than about 80 kDa and without a free sulfhydryl group.

8. The drug-linker-albumin of claim 7, wherein the therapeutic drug molecule is selected from the group consisting of: lactoferrin, a growth hormone, insulin, a parathyroid hormone (PTH), teriparatide (or FORTEO^{™}), amphotericin B and a chemical drug derivative thereof.

9. The drug-linker-albumin of claim 1, wherein the heterobifunctional linker comprises a 6-maleimidohexanoic acid N-hydroxysuccinimide ester.

10. A method of preparing the drug-linker-albumin conjugate of claim 1, comprising the following steps of:
(1) covalently coupling a therapeutic drug molecule to one end of a heterobifunctional linker, thereby generating a therapeutic drug-linker conjugate;
(2) removing, or substantially removing, excess uncoupled heterobifunctional linker by desalting or chemical separation, thereby obtaining a preparation comprising a therapeutic drug-linker conjugate having no or substantially no excess uncoupled heterobifunctional linker;
(3) adding a recombinant human serum albumin to the preparation under conditions wherein the recombinant human serum albumin chemically couples to the therapeutic drug-linker conjugate, thereby generating a therapeutic drug-linker-albumin conjugate.

11. The method of claim 10, wherein the heterobifunctional linker comprises 6-maleimidohexanoic acid N-hydroxysuccinimide ester.

12. The method of claim 10 or claim 11, wherein the therapeutic drug reacts with the heterobifunctional linker at a molecular molar ratio of 1:5 in step (1), and in step (2) the recombinant human serum albumin reacts with the therapeutic drug-linker conjugate at a molecular molar ratio of 1:(0.2-1).
